# EUROPEAN PATENT APPLICATION

(11) **EP 4 218 550 A1**
(43) Date of publication of application: **02.08.2023**
(21) Application number: 23154616.9
(22) Date of filing: 27.02.2018
(51) Int. Cl.: A61B 5/00, A61B 5/145, A61B 5/11

(54) **SYSTEMS, DEVICES, AND METHODS FOR WELLNESS AND NUTRITION MONITORING AND MANAGEMENT USING ANALYTE DATA**

(30) Priority: 08.03.2017 US 201762468843 P
(62) Divisional of application: 18712038.1
(71) Applicant: Abbott Diabetes Care Inc., Alameda, California 94502 (US)
(72) Inventor: COLE, Jean-Pierre, Tracy, CA 95304 (US); FELDMAN, Benjamin J., Berkeley, CA 94704 (US); HOSS, Udo, Castro Valley, CA 94552 (US); KIAIE, Namvar, Danville, CA 94506 (US); ARBOGAST, Frederic T., Newtown, PA 18940 (US)
(74) Representative: Booth, Catherine Louise

(57) **Abstract**

Systems, devices and methods are provided for the monitoring and management of an individual's wellness and nutrition using analyte data from an in vivo analyte sensor. Generally, a sensor control device is provided for wear on the body. The sensor control device can include an in vivo analyte sensor for measuring an analyte level in a bodily fluid, an accelerometer for measuring the physical activity level of the subject, as well as communications circuitry for wirelessly transmitting data to a reader device. Furthermore, disclosed herein are embodiments of various graphical user interfaces for displaying analyte metrics on a reader device, comparing the analyte response of various foods and/or meals, modifying daily nutrient recommendations based on analyte metrics and physical activity level measurements, and other features described herein. Additionally, the embodiments disclosed herein can be used to monitor various types of analytes.

## Description

### FIELD

The subject matter described herein relates generally to systems, devices, and methods for the monitoring and managing of an individual's wellness and nutrition based at least in part on analyte data from an in vivo analyte sensor.

### BACKGROUND

The monitoring and management of wellness and nutrition in individuals can significantly benefit those at risk of or currently experiencing chronic health problems and those motivated to improve general wellness. These efforts can create several health and economic benefits for the individual, as well as the public at large. According to the CDC, for example, seven out often deaths in the United States occur each year from chronic diseases, and almost one out of every two adults has at least one chronic illness. Likewise, almost one in three children in the United States is overweight or obese, which predisposes them to chronic diseases. Many of these chronic diseases are preventable, or can be successfully treated if diagnosed at an early stage. In this regard, the monitoring and management of an individual's wellness and nutrition can significantly reduce the chance of chronic disease and, as a result, can mitigate future healthcare costs. Additional benefits of wellness and nutrition monitoring can further include enhancing athletic performing either during training, recovery, or during an athletic event.

To promote these goals, wearable technology (e.g., Fitbit) can be utilized. A compact electronic device, for example, may be worn on the body, such as around the wrist, for monitoring an individual's heart rate or physical activity levels. Because physician visits are episodic (e.g., once per year), wearable technology can serve a useful function in providing timely physiological information to an individual, without the need for a physician visit, and which can ultimately lead to improved wellness. Despite these advantages, however, many people are reluctant to use wearable technology for various reasons, including the complexity of the data presented, a learning curve associated with using the wearable device, and inaccuracies with respect to the data. Some recent studies, for example, claim that existing wearable devices do not accurately measure an individual's heart rate or the number of calories burned.

Thus, needs exist for systems, devices and methods for wellness and nutritional monitoring and management that are more accurate and simpler to use by an individual.

### SUMMARY

Provided herein are example embodiments of systems, devices and methods for monitoring and managing the wellness and nutrition of an individual based at least in part on analyte data received from an in vivo analyte sensor. Generally, a sensor control device with a small form factor can be provided to an individual to wear on their body. The sensor control device can include, within a single housing, an in vivo analyte sensor for measuring an analyte level (or multiple analyte levels) in a subject, and an accelerometer for measuring the activity level of the subject. The in vivo analyte sensor can be configured such that at least a portion of the sensor is in contact with a bodily fluid of the subject. The sensor control device can also include communications circuitry for wirelessly transmitting data to a reader device. The sensor control device can be designed as a consumer-grade product. In those embodiments, for privacy, security and regulatory reasons, the raw analyte level measurements are not stored in the memory of the sensor control device, nor is such data encrypted or made accessible to the individual.

The reader device, which receives data from the sensor control device, can be a smart phone, tablet computer, personal digital assistant or other proprietary or non-proprietary mobile computing platform. One or more applications can be installed on the reader device, which analyzes data transmitted from the sensor control device and displays information relating to wellness and nutrition to the individual. In some embodiments, for example, a simple carbohydrate graph is displayed without showing the values of the underlying analyte data. Instead, different colored bars can indicate various analyte levels. In other embodiments, a numerical score representing an analyte response for an ingested food or meal is displayed. In still other embodiments, a daily nutrient recommendation based on physical activity level data can be displayed to the individual. These embodiments and others described herein are improvements in the field of computer-based wellness and nutrition monitoring over prior or existing wearable devices. Other improvements and advantages are provided, and will be apparent to those of skill in the art. The various configurations of these devices are described by way of the embodiments which are only examples.

Other systems, devices, methods, features and advantages of the subject matter described herein will be or will become apparent to one with skill in the art upon examination of the following figures and detailed description. It is intended that all such additional systems, devices, methods, features and advantages be included within this description, be within the scope of the subject matter described herein, and be protected by the accompanying claims. In no way should the features of the example embodiments be construed as limiting the appended claims, absent express recitation of those features in the claims.

### BRIEF DESCRIPTION OF THE FIGURES

The details of the subject matter set forth herein, both as to its structure and operation, may be apparent by study of the accompanying figures, in which like reference numerals refer to like parts. The components in the figures are not necessarily to scale, emphasis instead being placed upon illustrating the principles of the subject matter. Moreover, all illustrations are intended to convey concepts, where relative sizes, shapes and other detailed attributes may be illustrated schematically rather than literally or precisely.
FIG. 1 is a system overview of a sensor control device, reader device, network, local computer system and trusted computer system.
FIG. 2 is a block diagram depicting an example embodiment of a reader device.
FIGS. 3A and 3B are block diagrams of example embodiments of sensor control devices.
FIG. 4 is an example graph indicating multiple analyte curves over time.
FIG. 5 is another example graph indicating multiple analyte curves over time.
FIG. 6 is an example embodiment of a graphical user interface for displaying analyte metrics on a reader device.
FIG. 7 is an example embodiment of a graphical user interface for analyzing food impact.
FIG. 8 is an example embodiment of a graphical user interface for displaying an individual's glucose tolerance.
FIG. 9 is another example embodiment of a graphical user interface depicting a graph for monitoring a subject's carbohydrate intake.

### DETAILED DESCRIPTION

Before the present subject matter is described in detail, it is to be understood that this disclosure is not limited to the particular embodiments described herein, as such may, of course, vary. It is also to be understood that the terminology used herein is for the purpose of describing particular embodiments only, and is not intended to be limiting, since the scope of the present disclosure will be limited only by the appended claims.

As used herein and in the appended claims, the singular forms "a," "an," and "the" include plural referents unless the context clearly dictates otherwise.

The publications discussed herein are provided solely for their disclosure prior to the filing date of the present application. Nothing herein is to be construed as an admission that the present disclosure is not entitled to antedate such publication by virtue of prior disclosure. Further, the dates of publication provided may be different from the actual publication dates which may need to be independently confirmed.

Generally, embodiments of the present disclosure include systems, devices, and methods for monitoring and managing the wellness and nutrition of an individual based at least in part on analyte data from an in vivo analyte sensor. Accordingly, many embodiments include in vivo analyte sensors configured so that at least a portion of the sensor is, or can be, positioned in the body of a user to obtain information about at least one analyte of the body, such as glucose, in a bodily fluid (e.g., subcutaneously within the interstitial fluid ("ISF") or blood, within the dermal fluid of the dermal layer, or otherwise). In some embodiments, for example, the sensor is configured to measure a glucose level. In other embodiments, the sensor can be configured to measure a ketone level instead of, or in addition to, measuring a glucose level. Additionally, the detection of other analytes is within the scope of the present disclosure, and can include, for example, lactate, oxygen, hemoglobin A1C, acetyl choline, amylase, bilirubin, cholesterol, chorionic gonadotropin, creatine kinase (e.g., CK-MB), creatine, DNA, fructosamine, glutamine, growth hormones, hormones, peroxide, prostate-specific antigen, prothrombin, RNA, thyroid stimulating hormone, troponin and others. The embodiments disclosed herein can also be used with in vivo analyte monitoring systems that incorporate in vitro capability, as well as purely in vitro or ex vivo analyte monitoring systems, including systems that are entirely non-invasive.

Furthermore, for each and every embodiment of a method disclosed herein, systems and devices capable of performing each of those embodiments are covered within the scope of the present disclosure. For example, embodiments of sensor control devices are disclosed and these devices can have one or more sensors, accelerometers, analyte monitoring circuits (e.g., an analog circuit), non-transitory memories (e.g., for storing instructions and data), power sources, communication circuits, transmitters, receivers, processors and/or controllers (e.g., for executing instructions stored in memory) that can perform any and all method steps, or facilitate the execution of any and all method steps.

A number of embodiments of the present disclosure are designed to improve upon the accuracy and ease-of-use with respect to wearable technology through the use of analyte data received from an in vivo analyte sensor. In some embodiments, for example, a sensor control device is worn on the body, where the sensor control device includes an in vivo analyte sensor and an accelerometer. Analyte metrics are determined by one or more processors of the sensor control device, and transmitted, along with physical activity level measurements, to a reader device. Analyte level measurements on the sensor control device can subsequently be discarded. In other words, the sensor control device can be specifically configured not to transmit analyte level measurements. At the reader device, various information is presented on the display. In some embodiments, for example, a simple carbohydrate graph can be displayed to the user with colored bars indicating different ranges of analyte levels. In other embodiments, a numerical score can be presented to the user, with the score reflecting an analyte response to an ingested food or meal. In still other embodiments, analyte metrics along with physical activity level measurements can be analyzed to determine whether a daily nutrient recommendation needs to be adjusted. Accordingly, these embodiments can provide a subject with accurate, customizable, easy-to-use and easily understood information regarding wellness and/or nutrition, based on analyte data received from an in vivo analyte sensor. The disclosed embodiments can improve upon the accuracy of prior systems in that actual analyte level measurements are utilized and, in some embodiments, corroborated with physical analyte level measurements from an accelerometer. In addition, the disclosed embodiments can improve upon user adherence to wellness and nutrition monitoring regimens by presenting a simple, easy-to-use interface. Other features and advantages of the disclosed embodiments are further discussed below.

Before describing these aspects of the embodiments in detail, however, it is first desirable to describe examples of devices that can be present within, for example, a sensor control device that transmits data from an in vivo analyte sensor, as well as examples of their operation, all of which can be used with the embodiments described herein.

There are various types of systems which utilize in vivo analyte sensors. "Continuous Analyte Monitoring" systems (e.g., "Continuous Glucose Monitoring" systems), for example, can transmit data from a sensor control device to a reader device continuously or repeatedly with or without prompting, e.g., automatically according to a schedule. "Flash Analyte Monitoring" systems (e.g., "Flash Glucose Monitoring" systems or simply "Flash" systems), as another example, can transfer data from a sensor control device in response to a user-initiated request for data by a reader device (e.g., a scan), such as with a Near Field Communication (NFC) or Radio Frequency Identification (RFID) protocol. In vivo analyte monitoring systems can also operate without the need for finger stick calibration.

In vivo analyte monitoring systems can be differentiated from "in vitro" systems that contact a biological sample outside of the body (or rather "ex vivo") and that typically include a meter device that has a port for receiving an analyte test strip carrying bodily fluid of the user, which can be analyzed to determine the user's blood sugar level.

In vivo monitoring systems can include a sensor that, while positioned in vivo, makes contact with the bodily fluid of the user and senses the analyte levels contained therein. The sensor can be part of the sensor control device that resides on the body of the user and contains the electronics and power supply that enable and control the analyte sensing. The sensor control device, and variations thereof, can also be referred to as a "sensor control unit," an "on-body electronics" device or unit, an "on-body" device or unit, or a "sensor data communication" device or unit, to name a few.

In vivo monitoring systems can also include a reader device that receives sensed analyte data from the sensor control device, processes and/or displays that sensed analyte data, in any number of forms, to the user. This device, and variations thereof, can be referred to as a "handheld reader device," "reader device" (or simply a "reader"), "handheld electronics" (or simply a "handheld"), a "portable data processing" device or unit, a "data receiver," a "receiver" device or unit (or simply a "receiver"), or a "remote" device or unit, to name a few. Other devices such as personal computers, smartphones and similar devices have also been utilized with or incorporated into in vivo and in vitro monitoring systems.

### Example Embodiments of Individual Wellness and Nutrition Monitoring Systems

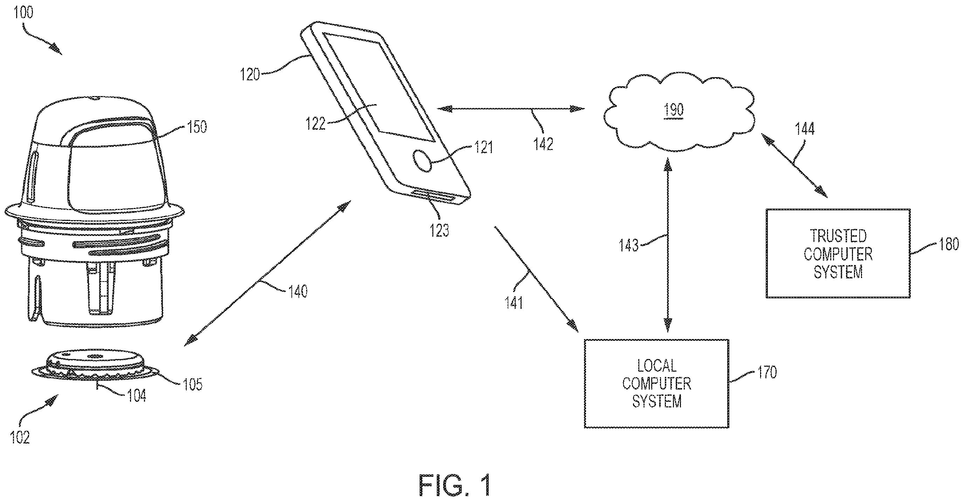

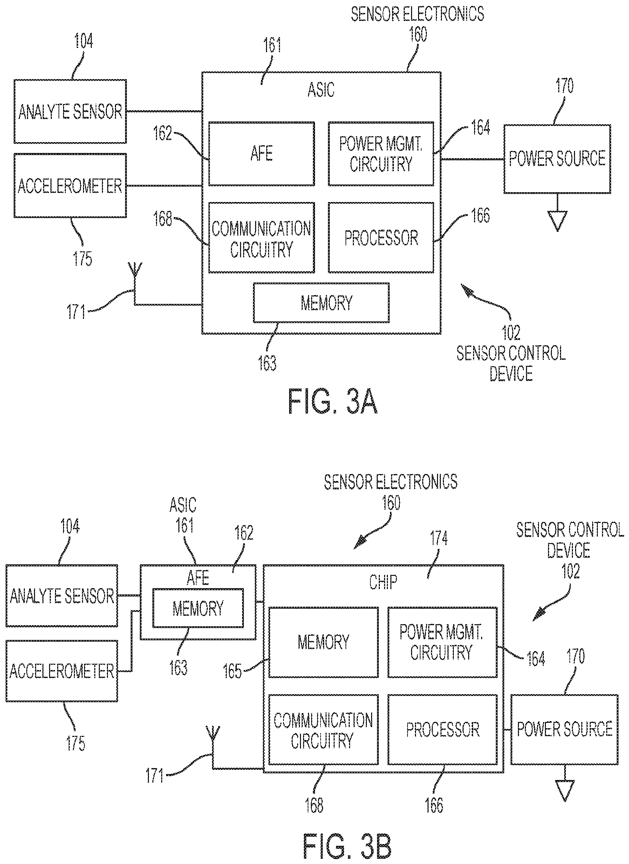

FIG. 1 is a conceptual diagram depicting an example embodiment of a wellness and nutrition monitoring system 100 that includes a sensor control device 102 and a reader device 120. System 100 can also include sensor applicator 150, which can be used to apply sensor control device 102 to a monitoring location on a user's skin such that a sensor 104 is maintained in position in the user's body for a period of time by an adhesive patch 105. Sensor control device 102 is further described with respect to FIGS. 3A and 3B, and can communicate with reader device 120 via a communication path 140 using a wired or wireless technique. Example wireless protocols include Bluetooth, Bluetooth Low Energy (BLE, BTLE, Bluetooth SMART, etc.), Near Field Communication (NFC), Wi-Fi, and others.

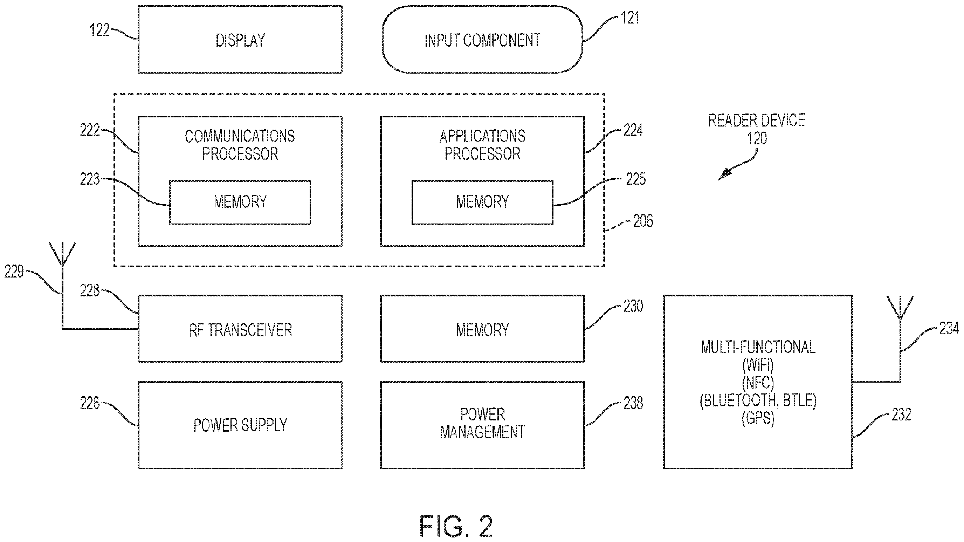

Individuals can monitor and use one or more wellness and nutrition applications installed in memory on reader device 120 using screen 122 and input 121, and the reader device battery can be recharged using power port 123. More details about reader device 120 are set forth with respect to FIG. 2 below. Reader device 120 can communicate with local computer system 170 via a communication path 141 using a wired or wireless technique. Local computer system 170 can include one or more of a laptop, desktop, tablet, phablet, smartphone, set-top box, video game console, or other computing device and wireless communication can include any number of applicable wireless networking protocols including Bluetooth, Bluetooth Low Energy (BTLE), Wi-Fi or others. Local computer system 170 can communicate via communications path 143 with a network 190, similar to how reader device 120 can communicate via a communications path 142 with network 190 by wired or wireless technique as described previously. Network 190 can be any of a number of networks, such as private networks and public networks, local area or wide area networks, and so forth. Network 190 can be the cloud. A trusted computer system 180 can include a server and can provide authentication services and/or secured data storage and can communicate via communications path 144 with network 190 by wired or wireless technique. Trusted computer system 180 can be considered part of network 190 (or the cloud) when considered from the perspective of devices 102, 120, and 170.

### Example Embodiments of Reader Devices

FIG. 2 is a block diagram depicting an example embodiment of a reader device 120 configured as a smartphone. Here, reader device 120 can include a display 122, input component 121, and processing circuitry 206, including a communications processor 222 coupled with non-transitory memory 223 and an applications processor 224 coupled with non-transitory memory 225. Also included can be separate non-transitory memory 230, RF transceiver 228 with antenna 229, and power supply 226 with power management module 238. Further included can be a multi-functional transceiver 232 which can communicate over Wi-Fi, NFC, Bluetooth, BTLE, ANT+, and GPS networks with an antenna 234. As understood by one of skill in the art, these components are electrically and communicatively coupled in a manner to make a functional device.

In addition, though described here as a smartphone, reader device 120 can also be a mobile smart wearable electronics assembly, such as an optical assembly that is worn over or adjacent to the user's eye (e.g., a smart glass or smart glasses, such as Google glasses, which is a mobile communication device). This optical assembly can have a transparent display that displays information about the user's analyte level (as described herein) to the user while at the same time allowing the user to see through the display such that the user's overall vision is minimally obstructed. The optical assembly may be capable of wireless communications similar to a smart phone. Other examples of wearable electronics include devices that are worn around or in the proximity of the user's wrist (e.g., a watch, etc.), neck (e.g., a necklace, etc.), head (e.g., a headband, hat, etc.), chest, or the like. Similarly, reader device 120 can also be a tablet computer, personal digital assistant, laptop or any other mobile computing device or personal computing device. Reader device 120 can also be an enhancement and/or add-on to devices used to monitor activity or sports performance, such as devices configured to measure and/or display power, cadence, pace, heart rate, speed. It is fully within the scope of the embodiments described herein that such devices can be adapted to display analyte metrics.

### Example Embodiments of Sensor Control Devices

FIGS. 3A and 3B are block diagrams depicting example embodiments of sensor control devices 102 each including an analyte sensor 104, accelerometer 175 and sensor electronics 160 (including analyte monitoring circuitry) that, collectively, can have the majority of the processing capability for rendering end-result data, such as analyte metrics, which are suitable for display to the user. In FIG. 3A, a single semiconductor chip 161 is depicted that can be a custom application specific integrated circuit (ASIC). Shown within ASIC 161 are certain high-level functional units, including an analog front end (AFE) 162, power management (or control) circuitry 164, processor 166, and communication circuitry 168 (which can be implemented as a transmitter, receiver, transceiver, passive circuit, or otherwise according to the communication protocol). In this embodiment, both AFE 162 and processor 166 are used as in vivo analyte monitoring and accelerometer monitoring circuitry, but in other embodiments either circuit can perform the monitoring functions. Processor 166 can include one or more processors, microprocessors, controllers, and/or microcontrollers, each of which can be a discrete chip or distributed amongst (and a portion of) a number of different chips.

Accelerometer 175 can include one or more piezoelectric, piezoresistive and/or capacitive materials (e.g., lead zirconate titanate, quartz, or tourmaline), which are used to convert a mechanical motion and/or changes in velocity into electrical signals. Accelerometer 175 can also include small micro electro-mechanical systems (MEMS), and can have a single axis or multiple axes configuration, including a three-axis configuration.

A non-transitory memory 163 is also included within ASIC 161 and can be shared by the various functional units present within ASIC 161, or can be distributed amongst two or more of them. Memory 163 can also be a separate chip. Memory 163 can be volatile and/or non-volatile memory. In this embodiment, ASIC 161 is coupled with power source 170, which can be a coin cell battery, or the like. AFE 162 interfaces with in vivo analyte sensor 104 and accelerometer 175, and receives measurement data therefrom and outputs the data to processor 166 in digital form. Processor 166, in turn, can execute one or more instructions stored in memory 163, which can cause processor 166 to process the data to determine one or more analyte metrics (e.g., analyte curves, analyte curve profiles (e.g., area, slope and/or length), analyte rates of change), as well as physical activity level values, trend values, etc. This data can then be provided to communication circuitry 168 for sending, by way of antenna 171, to reader device 120 (not shown), for example, where additional processing may be needed by the resident software application to display the data. Only analyte metrics (including aggregated data and/or averaged data) are stored in memory 163, whereas raw analyte level measurements are subsequently discarded. For example, in some embodiments, raw analyte data reflective of glucose levels are not stored in persistent memory 163 on the sensor, and would therefore be inaccessible by any means (e.g., by a reader device). In other embodiments, raw analyte data reflective of glucose levels can be temporarily stored in RAM (random access memory), or another similar type of volatile memory. In these embodiments, raw analyte level measurements can be used to calculate analyte metrics, e.g., running average glucose level over the past forty-eight hours, after which the raw analyte measurements can be deleted or discarded.

FIG. 3B is similar to FIG. 3A but instead includes two discrete semiconductor chips 162 and 174, which can be packaged together or separately. Here, AFE 162 is resident on ASIC 161. As shown here, AFE 162 is coupled to both analyte sensor 104 and accelerometer 175. Referring to chip 174, processor 166 is integrated with power management circuitry 164 and communication circuitry 168 on chip 174. AFE 162 includes memory 163 and chip 174 includes memory 165, which can be isolated or distributed within. In one example embodiment (not shown), AFE 162 is combined with power management circuitry 164 and processor 166 on one chip, while communication circuitry 168 is on a separate chip. In another example embodiment (also not shown), both AFE 162 and communication circuitry 168 are on one chip, and processor 166 and power management circuitry 164 are on another chip. It should be noted that other chip combinations are possible, including three or more chips, each bearing responsibility for the separate functions described, or sharing one or more functions for fail-safe redundancy.

In some embodiments, sensor control device 102 collects raw measurement data from the body and transmits that raw data (with or without signal conditioning, and with or without other data such as temperature data) to reader device 120 for further algorithmic processing into a form representative of the wearer's analyte levels, which can then be displayed (or made displayable) by reader device 120. In other embodiments, that algorithmic processing is performed by sensor control device 102 prior to transmission to reader device 120.

### Example Analyte Level Measurements and Metrics

As previously described, in vivo analyte sensor 104 can be configured to measure the level of one or more analytes (e.g., glucose) in a bodily fluid (e.g., dermal fluid, interstitial fluid, subcutaneous fluid, or blood) of the subject. A typical glucose profile in a healthy subject shows relatively flat glucose levels with "peaks," or analyte curves, associated with meals. The size and shape of the analyte curves can be related to the amount and type of food ingested.

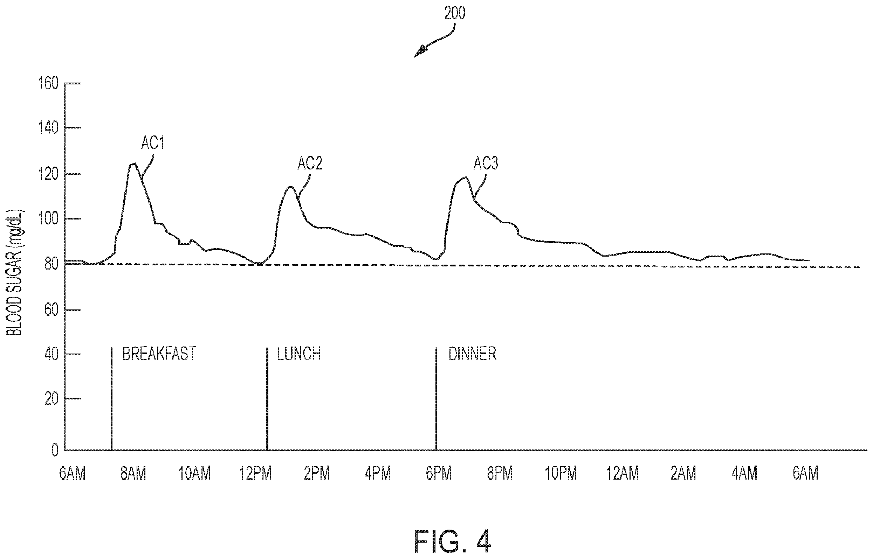

As one example, FIG. 4 is a graphical representation of analyte level measurements taken by analyte sensor 104, depicting a non-diabetic subject's glucose level over time. In particular, FIG. 4 shows graph 200 representative of a typical subject's analyte levels in response to three meals (e.g., breakfast, lunch and dinner) over a twenty-four hour period. A horizontal dashed line indicates a reference analyte level of the subject, such as a fasting blood glucose level. Analyte curve, AC1, depicts a rise in blood sugar level at approximately 7:30 AM in response to the subject's ingestion of breakfast. Likewise, analyte curves, AC2 and AC3, depict a rise in blood sugar levels at 12 PM and 6 PM, respectively, in response to the subject's ingestion of lunch and dinner. Each analyte curve, AC1, AC2 and AC3, can have an analyte curve profile, where each profile includes, at least: an area under the analyte curve, as depicted in FIG. 4 by the shaded region bound between the analyte level measurement and the reference analyte level (e.g., fasting blood glucose level), an analyte curve slope, and an analyte curve length.

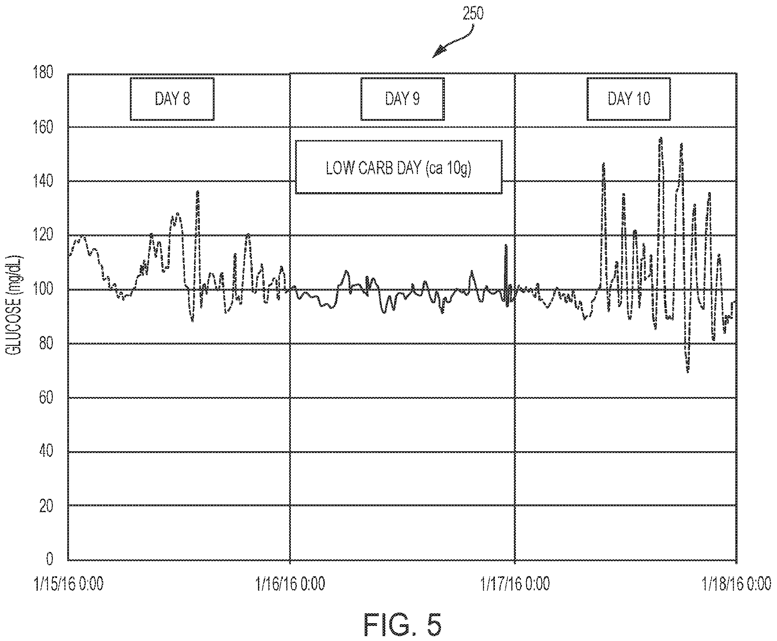

FIG. 5 is another graphical representation of analyte level measurements taken by analyte sensor 104 over three days. In particular, graph 250 in FIG. 5 reflects glucose data over a seventy-two hour period from a LIBRE sensor (manufactured by ABBOTT DIABETES CARE INC.) worn by a non-diabetic subject, representing Days 8-10 of a fourteen-day LIBRE sensor wear. On Day 9 (the central twenty-four hour period of the seventy-two hours shown in the graph), the non-diabetic subject consumed a low carbohydrate diet, with total ingested carbohydrates of about 10g. For the surrounding days (Days 8 and 10), a normal diet relatively high in carbohydrates was consumed. As evidenced by the smaller analyte curves, the glucose variability on Day 9 is lower, with the majority of raw glucose values falling between 90 and 110 mg/dL.

### Example Embodiments of Graphical User Interfaces for Displaying Analyte Metrics

Described herein are example embodiments of graphical user interfaces for displaying analyte metrics on reader device 120. As described above with respect to FIG. 4, in vivo analyte sensor 104 can measure analyte levels in a subject's bodily fluid and, by one or more sensor electronics processors, can determine one or multiple analyte metrics. Subsequently, the communications circuitry of sensor control device 102 can wirelessly transmit the analyte metrics to reader device 120. The raw analyte level measurements are subsequently discarded from sensor control device 102, as described earlier with respect to Figures 3A and 3B. In some embodiments, however, analyte metrics can be stored in memory on sensor control device 102 in an aggregated format. For example, in some embodiments, a running average of one or more analyte levels over a predetermined amount of time (e.g., past four hours) may be stored in memory of sensor control device 102. In other embodiments, a peak analyte level over a predetermined amount of time (e.g., 48 hours) can be stored in memory on sensor control device 102. These examples are meant to be illustrative, and not limiting in any sense, as those of skill in the art will readily understand that other types and formats of aggregated analyte metrics are within the scope of the disclosed embodiments.

Reader device 120 wirelessly receives the analyte metrics by its communications circuitry. Graphical user interfaces (GUIs), stored as instructions in the memory of reader device 120, are executed by the one or more reader device processors, and analyte metrics can be visually outputted to the display of reader device 120. In certain instances, as described below, the GUI is interactive, and a subject may enter information into reader device 120 through the GUI using an input device (e.g., a touch screen, a keyboard or mouse).

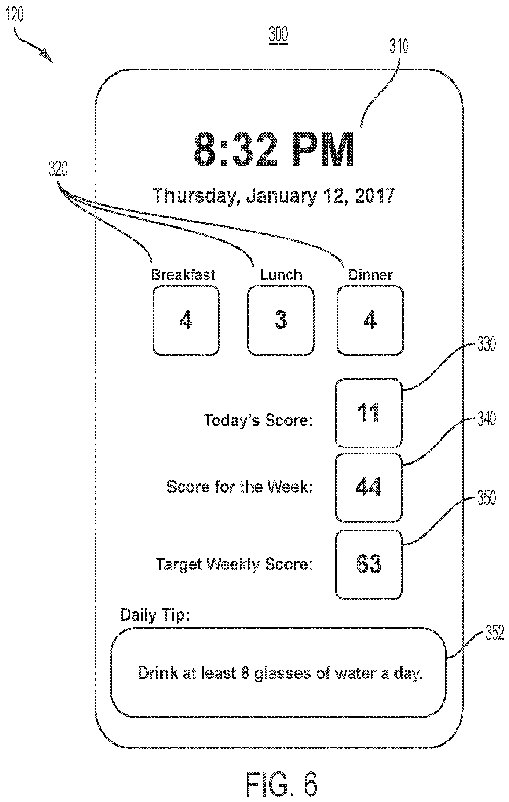

FIG. 6 is an example embodiment of a GUI 300 for displaying analyte metrics on reader device 120. Generally, GUI 300 can display simple, easy-to-read numerical scores which represent analyte curves associated with meals ingested by the subject, such as those described with respect to FIG. 4 (e.g., AC1, AC2, AC3). As shown in FIG. 6, for ease of reference, a date and time display 310 is depicted at a top portion of reader device 120. Below the date and time display 310, three numerical scores 320 in a horizontal orientation are shown, representing three meals ingested by the subject, i.e., breakfast, lunch and dinner. In some embodiments, the occurrence of meals can be determined by routines and/or algorithms stored in memory of reader device 120 and configured to detect patterns in the analyte metrics received from sensor control device 102. Detection of a meal event can include detection of analyte episodes or excursions outside a desired acceptable (e.g., medically recommended) target range in the user, who can be informed by the software that one or both has been detected. Examples of analyte excursions include violation of a low glucose threshold, violation of a high glucose threshold, violation of a rate of change (e.g., increase or decrease) threshold, violation of a glucose median threshold, violation of a glucose variability threshold, and the like. In other embodiments, the occurrence of meals can be further verified by routines configured to corroborate detected patterns in the analyte metrics with certain times of day. Other example embodiments of algorithms, routines or other sets of instructions for detecting meals and variations thereof are described in U.S. Patent Publication Nos. 2013/0085358, 2014/0350369 or 2014/0088393, or in Int'l Publ. No. WO 2015/153482, all of which are incorporated herein in their entirety and for all purposes. Some of the algorithms, routines or sets of instructions described in these incorporated references are described only in terms of identifying analyte excursions outside a desired target range. These embodiments can be extended to the detection of meal events based on the teachings contained within other ones of these incorporated references (e.g., Int'l Publ. No. WO 2015/153482). These embodiments can also be extended to the detection of meal events by specification of a within-target episode, where glucose values are maintained between an upper and lower bound for a period of time. Detection of these episodes can be done by extension of threshold-based episode detection algorithms.

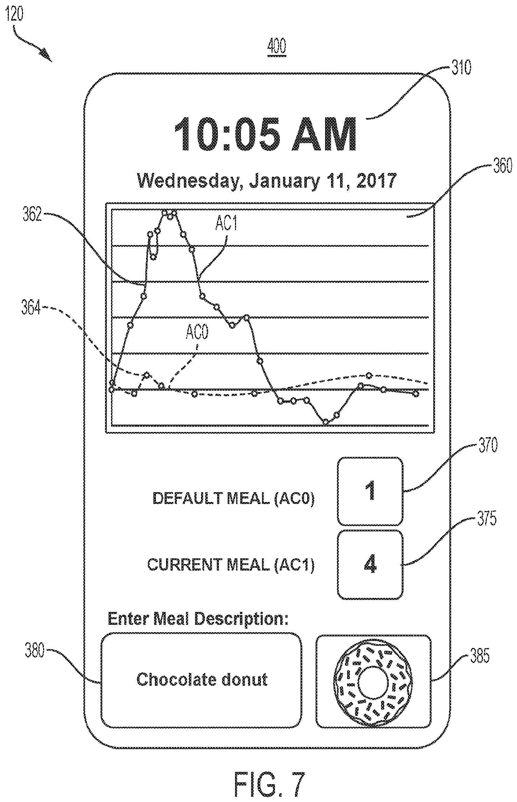

In still other embodiments, the occurrence of meals can be determined and/or verified manually by the user, for example, by prompting the user for input when a pattern is detected in analyte metrics received from sensor control device 102. For example, in some embodiments, the user can input a text-based description of the ingested meal into a text entry box using an input device of reader device 120, as further described with respect to FIG. 7. Each numerical score is proportional to an area under a corresponding analyte curve, such as those depicted in FIG. 4 (e.g., AC1, AC2, AC3). For simplicity and ease-of-reference, however, numerical scores, not analyte curves, are displayed in GUI 300.

Referring again to FIG. 6, the numerical score can be a whole number on a scale from one to five, with the whole number being proportional to an area under the analyte curve. A numerical score of three, for example, can reflect the area of a "default" analyte curve that represents an analyte response to a standard default meal. By contrast, a numerical score of five can indicate a food or meal that results in a relatively large analyte response, with an analyte curve having a greater area relative to the default meal. Conversely, a numerical score of one can indicate an ingested food or meal that results in a relatively small analyte response, with an analyte curve having a smaller area relative to the default meal. Moreover, as shown in FIG. 6, in many embodiments, the numerical scores reflect the areas underneath the one or more corresponding analyte curves. In other embodiments, however, a numerical score can also be a function of other metrics of an analyte curve profile, such as the slope of the analyte curve and/or the length of the analyte curve. In some embodiments, for example, the numerical score can be a function of the length of the analyte curve, where the length of the analyte curve is associated with the duration of an analyte response. The numerical score can also reflect an analyte curve profile for a specific type of food and/or meal. For example, in some embodiments, a relatively high numerical score can reflect an analyte curve characterized by a sharp spike, which can reflect a food or meal with refined carbohydrates (e.g., white-flour pasta). Conversely, in other embodiments, a relatively low numerical score can reflect an analyte curve characterized by a gradual slope, which can reflect a food or meal that is high in fiber or complex carbohydrates (e.g., whole-wheat pasta). Likewise, in other embodiments, the numerical score can also be a function of the rate of change of an analyte level.

Referring still to FIG. 6, three summary metrics 330, 340, 350 are displayed below the numerical scores 320 for breakfast, lunch and dinner. Today's Score 330 can indicate a sum of numerical scores for meals ingested on the current day. The Score for the Week 340 can indicate a sum of numerical scores for the week, up to the current day. The Target Weekly Score 350 can indicate a target numerical score which the subject should aspire to stay under. Additionally, as shown below the summary metrics, a daily tip or instruction 352 can be provided to help the subject to meet his or her Target Weekly Score. Thus, the simple GUI 300 presents easy-to-understand analyte metrics to the user, without a need for the subject to understand or interpret the underlying analyte level measurements acquired by sensor 104.

As shown in FIG. 6, in many embodiments, numerical scores can be shown as whole numbers on a scale from one to five. In other embodiments, however, numerical scores can be expressed as a percentage number that is greater than or less than the area under an analyte curve in response to the standard default food or meal (e.g., 30% above a standard, or 30% below a standard). In addition, in some embodiments, certain analyte metrics, such as Today's Score 330, the Score for the Week 340, or the Target Weekly Score 350, can be expressed as an average, instead of a sum, as shown in FIG. 6. In some embodiments, other non-numerical representations can be used in addition to, or in place of, the aforementioned numerical scores. For example, the use of different colored indicators (e.g., red light, yellow light or green light), textual indicators (e.g., good, neutral, bad), graphical indicators (thumbs up, thumbs down, happy face, sad face or other emoticons), letter grades (e.g., A, B, C, D or F), are all within the scope of the present disclosure.

Furthermore, because the relationship between an analyte curve and the ingested food or meal can be specific to each individual, the system can also be calibrated for each individual to an analyte curve for a standard default meal using a calibration feature (not shown). In some embodiments, calibration can be performed by a calibration feature, for example, by starting off with a standard default meal and adjusting the default analyte curve over time to the individual's average analyte curve. In some embodiments, the subject may decide which meal is representative for a standard meal and manually set the default response to that meal.

In addition, GUI 300 can include features to incentivize the subject to reach his or her Target Weekly Score 350. For example, in certain embodiments, GUI 300 can include a feature to send a message to the subject's friends through a social media platform (e.g., Facebook), to keep the subject's friends apprised of his or her status, or after a Target Weekly Score has been reached. In other embodiments, to encourage use of GUI 300, financial incentives can be provided (e.g., membership discounts), for initially signing up, or for referring friends who also sign-up to use the GUI. In other embodiments, upon reaching the Target Weekly Score, GUI 300 can provide the subject with one or more financial rewards such as discount codes, coupons, or in-app rewards.

### Example Embodiments of Graphical User Interfaces for Analyzing Food Impact

FIG. 7 is another example embodiment of a GUI for displaying analyte metrics on reader device 120. More specifically, GUI 400 can determine and display the physiological impact of a specific food or meal for a particular individual in comparison to a default standard food or meal. It is recognized that diet and nutrition must be customized for each individual. While it is true that there are common guidelines for all people, there is a wide variation in individual responses to foods based on individual physiology. Additionally, food choices may be restricted based on local availability, cultural acceptability, food allergies, and the like. GUI 400 can present information, specific to the individual, regarding which foods among those available provide the most nutritional benefit and the least negative impact on factors such as weight gain.

According to one aspect of the embodiment, reader device 120 wirelessly receives analyte metrics from sensor control device 102. As described earlier, the received analyte metrics can include an analyte curve profile for an ingested food or meal, wherein the analyte curve profile can include one or more of an area under the analyte curve, a slope of the analyte curve, and a length of the analyte curve. Subsequently, an application, in the form of instructions stored in memory of reader device 120, can be executed by the one or more reader device processors, causing the processors to associate a specific analyte curve profile for an ingested food or meal with a food entry in a database, and to store the analyte curve profile and the associated metrics in the database. The application can be further executed to perform a comparison between the analyte curve profile for the ingested food or meal with a default analyte curve profile for a standard default food or meal, and to visually output the results of the comparison to the display of reader device 120.

As shown in FIG. 7, GUI 400 includes graph 360 which depicts the physiological impact of a particular food or meal in comparison with a standard default food. In particular, analyte curve 362 for ingested meal or food, AC1, is shown in overlay with a default analyte curve 364, which reflects the subject's analyte response to the standard default meal or food, AC0. A date and time display 310 can also be included at a top portion of reader device 120. Numerical scores for the standard default meal or food 370 and the currently ingested meal 375, like the numerical scores described with reference to FIG. 6, can be displayed on GUI 400. Furthermore, in some embodiments, GUI 400 can also include a text entry box 380 and/or a pictorial entry box 385. The subject can input a text-based description of the ingested meal into text entry box 380 using the input device of reader device 120. The information entered can then be entered as a food entry into the database. Similarly, some embodiments can also include pictorial entry box 385, through which the subject can provide a photograph of the meal or food by using, for example, the camera on the reader device. The photograph can then be associated with the food entry and stored in the database.

In some embodiments, the food database can reside in memory on the reader device 120. In other embodiments, a database can be stored on a trusted computer system that is remote from reader device 120 and accessed over a local network, wide area network or the Internet. Additionally, GUI 400 can integrate with other third-party applications that facilitate the entry of food intake data, and their associated third-party food databases. In some embodiments, GUI 400 can also store and/or aggregate analyte metric data to a food database that can include data that is indicative of the impact of a specific food or meal on a population, or a portion of a population. Similarly, in some embodiments, GUI 400 can retrieve data from a food database regarding the impact of a specific food or meal for a population, or a portion of a population. The population data can reflect, for example, mean or median values, weighted averages, standard deviations and other statistical parameters for analyte metrics which can reflect the impact of a specific food or meal for a population (or portion thereof), and can be used to normalize and/or be compared to an individual's response to the same specific food or meal. In addition, population data collected from individual users can be made accessible to authorized individuals, groups, public health officials and/or payors.

Furthermore, in some embodiments, GUI 400 can also interface with one or more social media platforms, similar to those social media features described with respect to FIG. 6. For example, according to one aspect of the embodiment, GUI 400 can include a feature to send a message or notification to a subject's friends, to keep the subject's friends apprised of his or her status with respect to ingested meals, numerical scores associated with the ingested meals, or one or more indicators which reflect the impact of a specific ingested meal. In addition, in some embodiments, financial incentives can be provided (e.g., membership discounts, discount codes, in-app rewards), for initially signing up, or for referring friends who also sign-up to use the GUI.

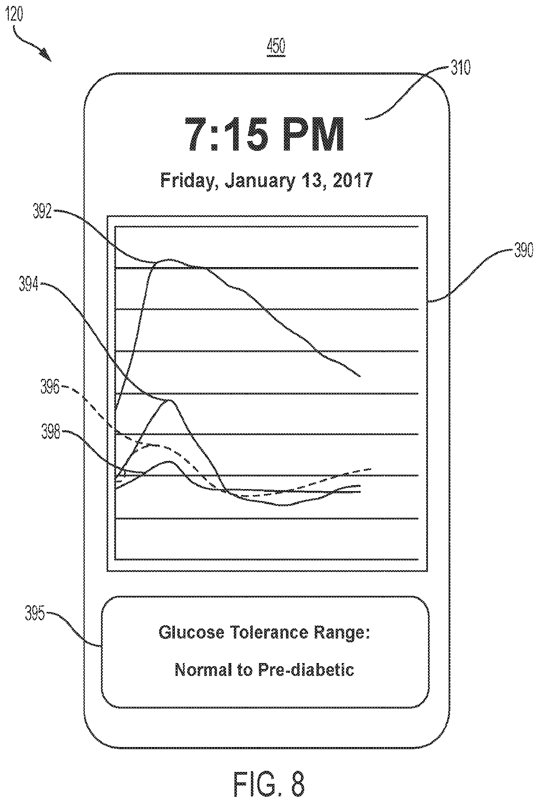

According to another aspect of the embodiment, GUI 450 can also assist in the detection of pre-diabetes. As shown in FIG. 8, chart 390 can initially include three analyte curves: (1) analyte curve 392, which represents a glucose response in a diabetic subject; (2) analyte curve 394, which represents a glucose response in a pre-diabetic subject; and (3) analyte curve 398, which represents a glucose response in a normal (non-diabetic and non pre-diabetic) subject. A dashed line, analyte curve 396, can represent a four-week average glucose response which is then superimposed with analyte curves 392, 394, 398. In this regard, the subject can visually approximate his or her analyte tolerance relative to normal, pre-diabetic and/or diabetic subjects. Additionally, a text display 395 can be provided under chart 390 to provide further guidance regarding the information displayed in chart 390.

According to other aspects of the embodiments, the GUIs described herein can also receive and process data from other types of sensors to provide an assessment of the health of the subject. For example, in some embodiments, other types of sensor data can include physical activity level measurements from the accelerometer on sensor control device 102. In other embodiments, other sensor data can include data from heart rate sensors, which can be incorporated into sensor control device 102, or deployed separately from sensor control device 102. Along with the GUIs, instructions stored in memory of reader device 120 can cause the one or more reader device processors to analyze the multiple types of sensor data to generate and display a comprehensive assessment of the subject's health.

### Example Embodiments of Graphical User Interfaces for Monitoring Carbohydrates

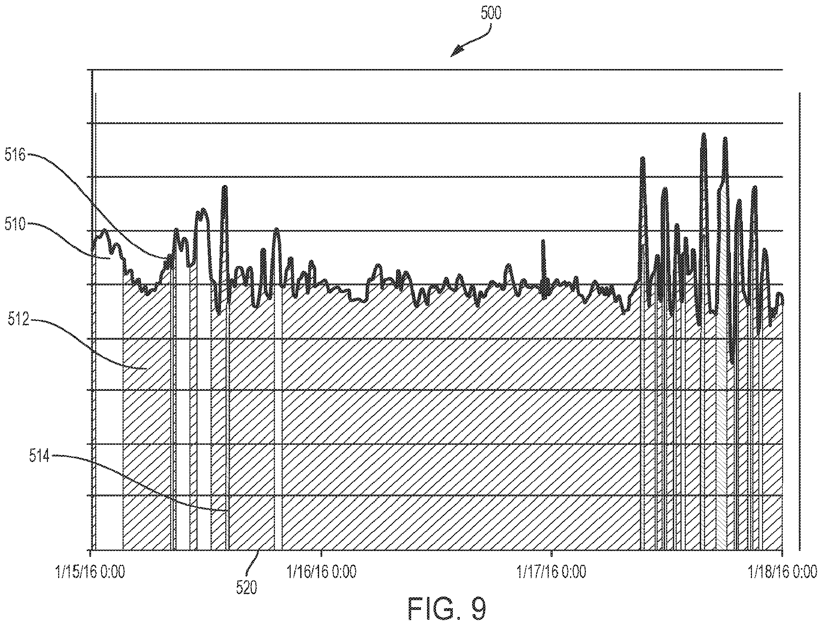

FIG. 9 is another example of a GUI for displaying analyte metrics on reader device 120. In particular, FIG. 9 shows a GUI 500 which includes a carbohydrate graph for monitoring a subject's carbohydrate input. It is generally understood that analyte metrics derived from a raw glucose signal, such as a signal acquired by an in vivo analyte sensor 104, can be representative of a subject's carbohydrate input. For example, analyte metrics reflecting a rate of change or integrated values above a certain threshold glucose value, may be more reflective of raw carbohydrate input than a raw glucose value. In this regard, carbohydrate graph of FIG. 9 reflects the subject's carbohydrate intake by displaying a graphical indication based on the one or more analyte metrics received from sensor control device 102. As shown in FIG. 9, the graphical indication can be a plotted line 516. In other embodiments, however, the graphical indication can also be a series of disconnected dots, an extrapolated line or curve, a scattershot, a shaded region, or other graphical representation of the received analyte metrics. In addition, as can be further seen in FIG. 9, horizontal axis 520 reflects increments of time units, spanning a seventy-two hour time window (e.g., from 1/15/16 0:00 to 1/18/16 0:00). It should be understood that other predetermined time windows can be utilized, e.g., between eight and ten hours, or between five and fifteen hours. The vertical axis is unlabeled and displays no values. GUI 500 is intuitive and easily comprehensible for the subject than, e.g., graph 250 of FIG. 5, since the subject may not have a technical understanding of numerical analyte levels necessary for the interpretation of raw analyte values.

Referring still to FIG. 9, plotted line 516 can include a series of plotted analyte values associated with the one or more of the metrics received from sensor control device 102. Furthermore, each plotted analyte value can be assigned to one or more ranges, such as a high range, a medium range or a low range, wherein each range can be represented by an indicator such as a colored band extending from the horizontal axis at the bottom portion of the graph up to the plotted value. For example, red-colored band 514 can indicate an analyte value assigned to a high range; yellow-colored band 510 can indicate an analyte assigned to a medium range; and green-colored band 512 can indicate an analyte value assigned to a low range. Although three ranges (high, medium and low) and three corresponding colors (red, green and yellow) are depicted in FIG. 9, other ranges, indicators, colors, visual patterns and gradations can be used, and are thus within the scope of this disclosure.

In some embodiments, each plotted analyte value can reflect an analyte level measurement acquired by in vivo analyte sensor 104. In other embodiments, however, the plotted analyte values can reflect different rates of change in the analyte level. Accordingly, a different color band can be assigned based on the integrated rate of change over a specified time period. For example, when the analyte level is changing more rapidly, the color band can be red. Likewise, when the rate of change decreases, the color band can be green.

According to another aspect of some embodiments, the assignment of plotted analyte values to a particular range and/or color band can also include the integration of past analyte values (or rates of change). For example, in some embodiments, if a predetermined number of preceding plotted analyte values are all assigned to a high range or, similarly, if there is a predetermined period of time in which all plotted analyte values are assigned to a high range, then the next plotted analyte value can also be assigned to a high range. Conversely, according to the same example embodiments, if a predetermined number of preceding plotted analyte values are all assigned to a low range or, similarly, if there is a predetermined period of time in which all plotted analyte values are assigned to a low range, then the plotted analyte value can also be assigned to a low range. In this regard, GUI 500 can include an "inertial resistance" algorithm in which if there is a prolonged period of low carbohydrate consumption, for example, a short period of high carbohydrate consumption would not necessarily cause the corresponding color bands to be red, as GUI 500 would "remember" the longer preceding integrated period of low-carbohydrate consumption. In some embodiments, the "inertial resistance" algorithm can be either activated or deactivated by the user through a settings interface of GUI 500 (not shown). Similarly, in some embodiments, the level of inertial resistance, i.e., the threshold duration or number of preceding, consecutive low-range (or high-range) analyte values, can be set by the user via an input device through the settings interface of GUI 500 (also not shown).

According to another aspect of the embodiment, GUI 500 can include multiple user-selected levels to control the "leniency" of the process which assigns the plotted analyte values to a particular range and/or color band. In particular, a subject can select either an "easy," "medium" or "hard" level through a settings interface (not shown) accessible through the input device of reader device 120. Instructions stored in memory on the reader device 120 cause the one or more reader device processors to determine the range of values for each of the low range, the medium range or the high range, based on the user-selected level. In other words, if the easy level is selected, the low range can include a greater range of values than either the medium range or the high range. If the medium level is selected, the medium range can include a greater or equal range of values than either the low range or the high range. If the hard level is selected, the high range can include a greater range of values than either the medium or the high range. In this regard, a subject may begin using GUI 500 at the easy setting, where many of the plotted analyte values are assigned to the low range and therefore have a green color. Thereafter, the subject may decide to advance to the medium or hard setting, in which fewer plotted analyte values are assigned to the low range, and "red" plotted analyte values are more prevalent. These settings allow the user to settle upon a level of feedback that both constructively incentivizes the user to improve, while also providing for the ability to discriminate between low and high carbohydrate diets.

According to another aspect of the embodiment, instructions stored in memory on sensor control device 102 and/or reader device 120 can be executed by one or more processors of the respective device to perform periodic monitoring (e.g., daily, weekly, monthly) of carbohydrate intake in order to establish a carbohydrate intake profile for a specific user. For example, in some embodiments, the instructions can cause the one or more processors to identify, store and recognize patterns in a user's carbohydrate intake. Based on identified patterns in the user's carbohydrate intake associated with specific foods and/or meals, a carbohydrate intake profile can be established. In some embodiments, the instructions can also detect analyte excursions that are inconsistent with an established carbohydrate intake profile, and subsequently prompt the user to input additional information regarding the types and amounts of foods and meals ingested by the user. These instructions can cause the processor to analyze the excursion and associated food inputs to determine the impact of different types and amounts of foods and meals. In other embodiments, the instructions can cause the processor to prompt the user for information regarding the type and/or amount of carbohydrates each time a food or meal is ingested. According to one aspect of these embodiments, the instructions can identify and/or store patterns in the user's carbohydrate intake profile after each food or meal is ingested. When a sufficient number of patterns have been detected, or after a predetermined period of time, the instructions can stop requiring user input each time a food and/or meal is ingested. In this regard, according to another aspect of the embodiment, the sensor control device and/or reader device can "learn" a particular user's carbohydrate intake patterns and predict an analyte response to a specific food or meal.

### Example Embodiments of Interfaces for Modifying Daily Nutrient Recommendations

In another example embodiment, a GUI is provided for generating and modifying a daily nutrient recommendation. The National Institutes of Health Food and Nutrition board of the U.S. Department of Health and Human Services has issued Dietary Reference Intake (DRI) documents to establish principles and guidelines of adequate dietary intakes. In particular, the Food and Nutrition Board renders authoritative judgments on the relationship among food intake, nutrition and health. Several interactive tools, such as the Interactive DRI for health care professionals provided by the U.S.D.A., can be used to calculate daily nutrient recommendations based on the DRI. The tools take into account gender, age, BMI and activity level to calculate: (1) estimated daily caloric need; (2) macronutrients such as carbohydrates, fiber, protein, fat and water; and (3) vitamins and minerals.

The following tables are provided as an example of a daily nutrient recommendation for a male subject that is 51 years old, having a height of 6' 1" and a weight of 170 lbs. In some embodiments, the daily nutrient recommendation shown below can be visually displayed on reader device 120. In particular, instructions stored in the memory of the reader device, when executed by the one or more reader device processors, can cause the one or more reader device processors to output the following information to the display of reader device 120.

| **You Entered:** | | | |
|---|---|---|---|
| **Male** | **Age:** | **Height:** | **Weight:** |
| | 51 years | 6 feet, 1 inch | 170 lbs. |
| Very Active | | | |

| **Results:** | | | |
|---|---|---|---|
| Body Mass Index (BMI) is 22.6 | | Estimated Daily Caloric Needs: 3473 kcal/day | |

| **Macronutrients:** Reference value refers to average daily nutrient intake; day-to-day nutrient intakes may vary. | |
|---|---|
| **Macronutrient** | **Recommended Intake per day** |
| Carbohydrate | 391 - 564 grams |
| Total Fiber | 30 grams |
| Protein | 62 grams |
| Fat | 77 - 135 grams |
| Saturated fatty acids | As low as possible while consuming a nutritionally adequate diet. |
| *Trans* fatty acids | As low as possible while consuming a nutritionally adequate diet. |
| a-Linolenic Acid | 1.6 grams |
| Linoleic Acid | 14 grams |
| Dietary Cholesterol | As low as possible while consuming a nutritionally adequate diet. |
| Total Water^{∗} | 3.7 Liters (about 16 cups) |

| | |
|---|---|
| ^{∗} Total water includes all water contained in food, beverages and drinking water. | |

| **Vitamins:** Reference value refers to *average daily nutrient intake*; day-to-day nutrient intakes may vary. | | |
|---|---|---|
| **Vitamin** | **Recommended Intake per day** | **Tolerable UL Intake per day** |
| Vitamin A | 900 meg | 3,000 mcg |
| Vitamin C | 90 mg | 2,000 mg |
| Vitamin D | 15 meg | 100 mcg |
| Vitamin B₆ | 2 mg | 100 mg |
| Vitamin E | 15 mg | 1,000 mg |
| Vitamin K | 120 meg | ND |
| Thiamin | 1 mg | ND |
| Vitamin B₁₂ | 2 meg | ND |
| Riboflavin | 1 mg | ND |
| Folate | 400 meg | 1,000 mcg |
| Niacin | 16 mg | 35 mg |
| Choline | 550 mg | 3,500 mg |
| Pantothenic Acid | 5 mg | ND |
| Biotin | 30 meg | ND |
| Carotenoids | NA | ND |

| **Minerals**: Reference value refers to average daily nutrient intake; day-to-day nutrient intakes may vary. | | |
|---|---|---|
| **Mineral** | **Recommended Intake per day** | **Tolerable UL Intake per day** |
| **Essential** | | |
| Calcium | 1,000 mg | 2,000 mg |
| Chloride | 2g | 3.6 g |
| Chromium | 30 mcg | ND |
| Copper | 900 mcg | 10,000 mcg |
| Fluoride | 4 mg | 10 mg |
| Iodine | 150 mcg | 1,100 mcg |
| Iron | 8 mg | 45 mg |
| Magnesium | 420 mg | 350 mg |
| Manganese | 2.3 mg | 11 mg |
| Molybdenum | 45 mcg | 2,000 mcg |
| Phosphorus | 700 mg | 4,000 mg |
| Potassium | 4.7 g | ND |
| Selenium | 55 mcg | 400 mcg |
| Sodium | 1.3 g | 2.3 g |
| Zinc | 11 mg | 40 mg |

| **Non-Essential** | | |
|---|---|---|
| Arsenic | NA | ND |
| Boron | NA | 20 mg |
| Nickel | NA | 1 mg |
| Silicon | NA | ND |
| Sulfate | NA | ND |
| Vanadium | NA | 1.8 mg |

According to one aspect of the embodiment, a GUI can be provided to: (1) monitor the individual subject's activity level to ensure that the personalized daily nutrient recommendation is accurate, and (2) monitor the individual's analyte response to correlate to carbohydrate intake. As described earlier, in addition to the in vivo analyte sensor 104, sensor control device 102 can also include accelerometer 175 for measuring a physical activity level in the subject. Furthermore, sensor control device 102 can wirelessly transmit one or more analyte metrics and physical level measurements to reader device 120. Subsequently, in some embodiments, instructions stored in memory of reader device 120, when executed by the one or more reader device processors, can cause the processors to determine, based on the one or more analyte metrics and the one or more physical activity level measurements, whether to adjust a daily nutrient recommendation.

According to another aspect of the embodiments, the subject's heart rate and daily heart rate patterns can be measured by a device, e.g., a heart rate monitor either incorporated into sensor control device 102 or deployed separately, and transmitted wirelessly to reader device 120. Subsequently, as with the analyte metrics and physical activity level measurements, the daily nutrient recommendation can be further modified based on one or more heart rate measurements.

Likewise, according to still another aspect of the embodiments, the subject's hydration level can be measured by a sensor device, either incorporated in sensor control device 102 or deployed separately, and transmitted wirelessly to reader device 120. Subsequently, as with the other physiological data, the daily nutrient recommendation can be further modified based on one or more hydration level measurements.

Similarly, according to yet another aspect of the embodiments, as described with reference to FIG. 9, the subject's glucose response can be correlated to the subject's carbohydrate intake. Accordingly, as with the other physiological data, the daily nutrient recommendation can be further modified based on the subject's carbohydrate intake.

Furthermore, according to another aspect of the embodiments, other parameters of the daily nutrient recommendation can be modified by the application based on feedback from any of the aforementioned sensor devices, including: an estimated daily caloric intake recommendation, a daily carbohydrate intake recommendation, a daily fiber intake recommendation, a daily protein intake recommendation, a daily fat intake recommendation, a daily water intake recommendation, a daily vitamin intake recommendation, and a daily mineral intake recommendation.

### Example Embodiments of Graphical User Interfaces for Ketone Monitoring

For all of the embodiments disclosed herein, references to analyte level measurements, analyte metrics, analyte curves and/or analyte curve profiles can refer to any number of analytes found in the bodily fluid of a subject, and which can be sensed by in vivo analyte sensor 104. In some embodiments, analyte metrics for ketone monitoring can be displayed to the subject on reader device 120. In particular, to assist a subject in maintaining a state of ketosis for dietary or medical reasons, a GUI can be provided for detecting when a ketosis threshold has not been met, and for displaying one or more recommendations to the subject for achieving the ketosis target threshold.

Several well-known diets, for example, are associated with decreasing carbohydrate intake (e.g., Atkins, Paleo, Ketogenic, Fasting). When a subject is on a carbohydrate- and/or a protein-restricted diet, the body must shift to using fat stores to generate energy. During this process, the body enters into nutritional ketosis, a state in which ketone bodies, including three compounds (i.e., acetone, acetoacetate, and the most prevalent, beta-hydroxybutyrates) can be detected in a bodily fluid. There is a general understanding that introduction of complex carbohydrates designed for slow metabolism can be introduced to the diet and still maintain a state of ketosis. In addition, monitoring ketones can be important to individuals with dietary needs or who suffer from epilepsy. A ketogenic diet is known to be an effective diet in reducing or eliminating the occurrence of seizures in people with epilepsy.

Prior or existing methods of determining whether or not a subject is in a state of nutritional ketosis are similar to those methods used by diabetics in identifying diabetic ketoacidosis. In both cases, urine strips (e.g., Ketostix) or blood-based strips (e.g., Abbott Diabetes Care Ketone Test Strips), can be used to determine a level of ketones in the body. However, urine-based testing provides a historic view of ketone levels with significant lag from the current status. While blood-based testing provides a more real-time result, the data set is episodic and dependent on how often the subject uses a test strip. This can result in lost information critical to understanding how food and exercise affect a person's state of nutritional ketosis.

The previously described embodiments can all be used for ketone monitoring either alone, or in conjunction with glucose monitoring. In some embodiments, for example, a GUI can be provided for reaching a Target Weekly Score with respect to ketone metrics (FIG. 6), or for determining a ketone response for a particular meal, food, supplement, or pharmaceutical (FIG. 7). Likewise, a daily nutrient recommendation for reaching and maintaining a desired state of ketosis can be determined (or modified) based on: (1) analyte metrics reflecting ketone levels in the subject's bodily fluid, and (2) physical activity level measurements from an accelerometer on sensor control device 102. According to another aspect of one embodiment, a GUI can be provided to determine and display one or more times of a day when a ketosis target threshold is not met, and to display one or more recommendations for achieving the ketosis target threshold. With respect to these embodiments and others, instructions can be stored in memory of reader device 120 that, when executed by one or more reader device processors, cause the one or more processors to analyze and display certain ketone metrics in order to assist the subject in reaching and maintaining a state of ketosis.

It should be noted that all features, elements, components, functions, and steps described with respect to any embodiment provided herein are intended to be freely combinable and substitutable with those from any other embodiment. If a certain feature, element, component, function, or step is described with respect to only one embodiment, then it should be understood that that feature, element, component, function, or step can be used with every other embodiment described herein unless explicitly stated otherwise. This paragraph therefore serves as antecedent basis and written support for the introduction of claims, at any time, that combine features, elements, components, functions, and steps from different embodiments, or that substitute features, elements, components, functions, and steps from one embodiment with those of another, even if the following description does not explicitly state, in a particular instance, that such combinations or substitutions are possible. It is explicitly acknowledged that express recitation of every possible combination and substitution is overly burdensome, especially given that the permissibility of each and every such combination and substitution will be readily recognized by those of ordinary skill in the art.

To the extent the embodiments disclosed herein include or operate in association with memory, storage, and/or computer readable media, then that memory, storage, and/or computer readable media are non-transitory. Accordingly, to the extent that memory, storage, and/or computer readable media are covered by one or more claims, then that memory, storage, and/or computer readable media is only non-transitory.

While the embodiments are susceptible to various modifications and alternative forms, specific examples thereof have been shown in the drawings and are herein described in detail. It should be understood, however, that these embodiments are not to be limited to the particular form disclosed, but to the contrary, these embodiments are to cover all modifications, equivalents, and alternatives falling within the spirit of the disclosure. Furthermore, any features, functions, steps, or elements of the embodiments may be recited in or added to the claims, as well as negative limitations that define the inventive scope of the claims by features, functions, steps, or elements that are not within that scope.

### Clauses

Clause 1. A system for displaying information relating to a subject's wellness and nutrition based at least in part on analyte data received from an in vivo analyte sensor, the system comprising: an in vivo analyte sensor configured to measure at least one analyte level in a bodily fluid; and sensor electronics configured to receive a signal indicative of one or more analyte level measurements from the in vivo analyte sensor, the sensor electronics comprising: one or more sensor electronics processors; an accelerometer coupled to the one or more sensor electronics processors, the accelerometer configured to measure a physical activity level of the subject; a memory coupled to the one or more sensor electronics processors, the memory storing instructions that, when executed by the one or more sensor electronics processors, cause the one or more sensor electronics processors to determine one or more analyte metrics based on the analyte level measurements, wherein the one or more analyte level measurements are discarded after the associated one or more analyte metrics are determined; and communications circuitry coupled to the one or more sensor electronics processors, the communications circuitry configured to wirelessly transmit at least one of the one or more analyte metrics and the one or more physical activity level measurements to a reader device.
Clause 2. The system of clause 1, wherein the one or more analyte metrics include an analyte curve and a numerical score associated with an area under the analyte curve, and wherein the analyte curve is associated with an ingested food or meal.
Clause 3. The system of clause 2, wherein the numerical score is a whole number within a predetermined scale, wherein the whole number is proportional to the area under the analyte curve.
Clause 4. The system of clause 3, wherein the predetermined scale is a range from 1 to 5, and wherein a numerical score of 3 reflects a default area under a default analyte curve in response to a standard default food or meal.
Clause 5. The system of clause 2, wherein the numerical score is a percentage number greater than or less than a default area under a default analyte curve in response to a standard default food or meal.
Clause 6. The system of clause 1, wherein the at least one analyte level comprises a glucose level.
Clause 7. The system of clause 2, further comprising a reader device, the reader device comprising: a display; communications circuitry adapted to wirelessly receive data indicative of the one or more analyte metrics; one or more reader device processors coupled to the display; and a memory coupled to the one or more reader device processors, the memory storing instructions that, when executed by the one or more reader device processors, cause the one or more reader device processors to output the one or more analyte metrics to the display, wherein the one or more analyte metrics include the numerical score.
Clause 8. The system of clause 7, wherein the instructions, when executed by the one or more reader device processors, further cause the one or more reader device processors to calculate and display a cumulative score based on the numerical scores received over a predetermined time period.
Clause 9. The system of clause 8, wherein the cumulative score is a sum of the numerical scores received over the predetermined time period.
Clause 10. The system of clause 8, wherein the cumulative score is an average of the numerical scores received over the predetermined time period.
Clause 11. The system of clause 8, wherein the instructions, when executed by the one or more reader device processors, further cause the one or more reader device processors to, if the cumulative score is below a predetermined target score at the end of the predetermined time period, send a message to one or more individuals affiliated with the subject through a social media platform, provide the subject with a discount code or coupon, or provide the subject with an in-app reward.
Clause 12. The system of clause 2, wherein the one or more analyte metrics further include an analyte curve profile for an ingested food or meal, the analyte curve profile comprising one or more of an area under an analyte curve, a slope of an analyte curve and a length of an analyte curve.
Clause 13. The system of clause 12, further comprising a reader device, the reader device comprising: a display; communications circuitry adapted to wirelessly receive data indicative of the one or more analyte metrics; one or more reader device processors coupled to the display; and a memory coupled to the one or more reader device processors, the memory storing instructions that, when executed by the one or more reader device processors, cause the one or more reader device processors to associate the analyte curve profile for an ingested food or meal with a food entry in a database, and to store the analyte curve profile in the database.
Clause 14. The system of clause 13, wherein the instructions, when executed by the one or more reader device processors, further cause the one or more reader device processors to compare the analyte curve profile for an ingested food or meal with a default analyte curve profile for a standard default food or meal, and to display the results of the comparison.
Clause 15. The system of clause 13, wherein the instructions, when executed by the one or more reader device processors, further cause the one or more reader device processors to display a graphical comparison between the analyte curve profile for an ingested food or meal with a default analyte curve profile for a standard default food or meal.
Clause 16. The system of clause 13, wherein the reader device further comprises an input device, and wherein the food entry includes information entered into the database by the subject using the input device.
Clause 17. The system of clause 13, wherein the food entry includes a picture of the ingested food or meal.
Clause 18. They system of clause 13, wherein the database resides on a trusted computer system remote from the reader device.
Clause 19. The system of clause 1, further comprising a reader device, the reader device comprising: a display; communications circuitry adapted to wirelessly receive data indicative of the one or more analyte metrics; one or more reader device processors coupled to the display; and a memory coupled to the one or more reader device processors, the memory storing instructions that, when executed by the one or more reader device processors, cause the one or more reader device processors to display a carbohydrate graph on the display, wherein the carbohydrate graph includes a graphical indication based on the one or more analyte metrics received from the sensor electronics over a predetermined time window, a horizontal axis reflecting increments in time units, and an unlabeled vertical axis.
Clause 20. The system of clause 19, wherein the graphical indication comprises a plurality of plotted analyte values associated with the one or more analyte metrics; wherein each plotted analyte value is assigned to one of a high range, a medium range, or a low range; and wherein either a first band corresponding to the high range, a second band corresponding to the medium range, or a third band corresponding to the low range extends from each plotted analyte value on the graphical indication down to a lower boundary of the carbohydrate graph.
Clause 21. The system of clause 20, wherein the first band is a red color, the second band is a yellow color and the third band is a green color.
Clause 22. The system of clause 19, wherein the predetermined time window is between 5 and 15 hours.
Clause 23. The system of clause 19, wherein the predetermined time window is 72 hours.
Clause 24. The system of clause 20, wherein each plotted analyte value comprises an analyte level measurement.
Clause 25. The system of clause 20, wherein each plotted analyte value comprises a rate of change of an analyte level.
Clause 26. The system of clause 20, wherein the instructions, when executed by the one or more reader device processors, further cause the one or more reader device processors to factor in a predetermined number of preceding plotted analyte values during assignment of each plotted analyte value to one of a high range, a medium range, or a low range, wherein if a predetermined number of preceding plotted analyte values are all assigned to a high range, then the plotted analyte value will also be assigned to the high range, and wherein if a predetermined number of preceding plotted analyte values are all assigned to a low range, then the plotted analyte value will also be assigned to the low range.
Clause 27. The system of clause 20, wherein the reader device further comprises an input device, wherein the instructions, when executed by the one or more reader device processors, further cause the one or more reader device processors to allow the subject to select one of a plurality of user-selected levels, wherein the user-selected levels include an easy level, a medium level and a hard level, wherein, if the easy level is selected, the low range includes a greater range of values than either the medium range or the high range, wherein, if the medium level is selected, the medium range includes a greater or equal range of values than either the low range or the high range, and wherein, if the hard level is selected, the high range includes a greater range of values than either the medium range or the high range.
Clause 28. The system of clause 12, wherein the instructions that, when executed by the one or more sensor electronics processors, further cause the one or more sensor electronics processors to determine a glucose tolerance based at least in part on the analyte curve profile.
Clause 29. The system of clause 1, further comprising a reader device, the reader device comprising: a display; communications circuitry adapted to wirelessly receive data indicative of the one or more analyte metrics and the one or more physical activity level measurements; one or more reader device processors coupled to the display; and a memory coupled to the one or more reader device processors, the memory storing instructions that, when executed by the one or more reader device processors, cause the one or more reader device processors to determine, based on the one or more analyte metrics and the one or more physical activity level measurements, whether to adjust a daily nutrient recommendation.
Clause 30. The system of clause 29, wherein the daily nutrient recommendation includes an estimated daily caloric intake recommendation.
Clause 31. The system of clause 29, wherein the daily nutrient recommendation includes at least one of a daily carbohydrate intake recommendation, a daily fiber intake recommendation, a daily protein intake recommendation, a daily fat intake recommendation, a daily water intake recommendation, a daily vitamin intake recommendation and a daily mineral intake recommendation.
Clause 32. The system of clause 29, wherein the daily nutrient recommendation is based at least in part on the subject's age, the subject's height, the subject's weight and the subject's body mass index.
Clause 33. The system of clause 29, wherein the communications circuitry is further adapted to receive data indicative of one or more heart rate measurements, and wherein the determination to adjust the daily nutrient recommendation is further based on the one or more heart rate measurements.
Clause 34. The system of clause 29, wherein the communications circuitry is further adapted to receive data indicative of one or more hydration level measurements, and wherein the determination to adjust the daily nutrient recommendation is further based on the one or more hydration level measurements.
Clause 35. The system of clause 1, wherein the at least one analyte level comprises a ketone level.
Clause 36. The system of clause 29, wherein the at least one analyte level comprises a ketone level.
Clause 37. The system of clause 36, wherein the instructions, when executed by the one or more reader device processors, further cause the one or more reader device processors to determine and display one or more times of a day when a ketosis target threshold is not met, and to display one or more recommendations for achieving the ketosis target threshold.
Clause 38. The system of clause 31, wherein the at least one analyte level comprises a ketone level and a glucose level, and wherein the instructions, when executed by the one or more reader device processors, further cause the one or more reader device processors to display a recommendation for a diet modification.
Clause 39. A method for monitoring and managing a subject's wellness and nutrition, the method comprising: measuring, by an in vivo analyte sensor, at least one analyte level in a bodily fluid; receiving, by sensor electronics, a signal indicative of one or more analyte level measurements, wherein the sensor electronics includes one or more sensor electronics processors, a memory, an accelerometer, and communications circuitry; measuring, by the accelerometer, a physical activity level of the subject; determining, by the one or more sensor electronics processors, one or more analyte metrics based on the analyte level measurements; after determining the one or more analyte metrics, discarding the analyte level measurements; and wirelessly transmitting at least one of the one or more analyte metrics and the one or more physical activity level measurements to a reader device.
Clause 40. The method of clause 39, wherein the one or more analyte metrics include an analyte curve and a numeral score associated with an area under the analyte curve, and wherein the analyte curve is associated with an ingested food or meal.
Clause 41. The method of clause 40, wherein the numerical score is a whole number within a predetermined scale, wherein the whole number is proportional to the area under the analyte curve.
Clause 42. The method of clause 41, wherein the predetermined scale is a range from 1 to 5, and wherein a numerical score of 3 reflects a default area under a default analyte curve in response to a standard default food or meal.
Clause 43. The method of clause 40, wherein the numerical score is a percentage number greater than or less than a default area under a default analyte curve in response to a standard default food or meal.
Clause 44. The method of clause 39, wherein the at least one analyte level comprises a glucose level.
Clause 45. The method of clause 40, further comprising: wirelessly receiving, by communications circuitry of a reader device, data indicative of the one or more analyte metrics, wherein the reader device comprises a display, one or more reader device processors, and a memory; outputting the one or more analyte metrics to the display, wherein the one or more analyte metrics include the numerical score.
Clause 46. The method of clause 45, further comprising calculating and displaying a cumulative score based on the numerical scores received over a predetermined time period.
Clause 47. The method of clause 46, wherein the cumulative score is a sum of the numerical scores received over the predetermined time period.
Clause 48. The method of clause 46, wherein the cumulative score is an average of the numerical scores received over the predetermined time period.
Clause 49. The method of clause 46, further comprising, if the cumulative score is below a predetermined target score at the end of the predetermined time period, sending a message to one or more individuals affiliated with the subject through a social media platform, providing the subject with a discount code or coupon, or providing the subject with an in-app reward.
Clause 50. The method of clause 40, wherein the one or more analyte metrics further include an analyte curve profile for an ingested food or meal, the analyte curve profile comprising one or more of an area under an analyte curve, a slope of an analyte curve and a length of an analyte curve.
Clause 51. The method of clause 50, further comprising: wirelessly receiving, by communication circuitry of a reader device, data indicative of the one or more analyte metrics, wherein the reader device comprises a display, one or more reader device processors, and a memory; associating the analyte curve profile for an ingested food or meal with a food entry in a database; and storing the analyte curve profile in the database.
Clause 52. The method of clause 51, further comprising: comparing the analyte curve profile for an ingested food or meal with a default analyte curve profile for a standard default food or meal; and displaying the results of the comparison.
Clause 53. The method of clause 51, further comprising displaying a graphical comparison between the analyte curve profile for an ingested food or meal with a default analyte curve profile for a standard default food or meal.
Clause 54. The method of clause 51, further comprising creating the food entry in the database, and entering information associated with the food entry into the database using an input device of the reader device.
Clause 55. The method of clause 51, wherein the food entry includes a picture of the ingested food or meal.
Clause 56. The method of clause 51, wherein the database resides on a trusted computer system remote from the reader device.
Clause 57. The method of clause 39, further comprising: wirelessly receiving, by communications circuitry of a reader device, data indicative of the one or more analyte metrics, wherein the reader device comprises a display, one or more reader device processors, and a memory; and displaying a carbohydrate graph on the display, wherein the carbohydrate graph includes a graphical indication based on the one or more analyte metrics received from the sensor electronics over a predetermined time window, a horizontal axis reflecting increments in time units, and an unlabeled vertical axis.
Clause 58. The method of clause 57, wherein the graphical indication comprises a plurality of plotted analyte values associated with the one or more analyte metrics, the method further comprising: assigning each plotted analyte value to one of a high range, a medium range or a low range; and for each plotted analyte value, displaying either a first band corresponding to the high range, a second band corresponding to the medium range, or a third band corresponding to the low range, extending from the plotted analyte value on the graphical indication down to a lower boundary of the carbohydrate graph.
Clause 59. The method of clause 58, wherein the first band is a red color, the second band is a yellow color, and the third band is a green color.
Clause 60. The method of clause 57, wherein the predetermined time window is between 5 and 15 hours.
Clause 61. The method of clause 57, wherein the predetermined time window is 72 hours.
Clause 62. The method of clause 58, wherein each plotted analyte value comprises an analyte level measurement.
Clause 63. The method of clause 58, wherein each plotted analyte value comprises a rate of change of an analyte level.
Clause 64. The method of clause 58, wherein assigning each plotted analyte value to one of a high range, a medium range or a low range further comprises factoring in a predetermined number of preceding plotted analyte values, wherein if a predetermined number of preceding plotted analyte values are all assigned to a high range, then the plotted analyte value will also be assigned to the high range, and wherein if a predetermined number of preceding plotted analyte values are all assigned to a low range, then the plotted analyte value will also be assigned to the low range.
Clause 65. The method of clause 58, further comprising selecting one of a plurality of user selected levels, wherein the user-selected levels include an easy level, a medium level and a hard level, wherein, if the easy level is selected, the low range includes a greater range of values than either the medium range or the high range, wherein, if the medium level is selected, the medium range includes a greater or equal range of values than either the low range or the high range, and wherein, if the hard level is selected, the high range includes a greater range of values than either the medium range or the high range.
Clause 66. The method of clause 50, further comprising determining a glucose tolerance based at least in part on the analyte curve profile.
Clause 67. The method of clause 39, further comprising: wirelessly receiving, by communications circuitry of a reader device, data indicative of the one or more analyte metrics and the one or more physical activity level measurements, wherein the reader device comprises a display, one or more reader device processors, and a memory; and determining, based on the one or more analyte metrics and the one or more physical activity level measurements, whether to adjust a daily nutrient recommendation.
Clause 68. The method of clause 67, wherein the daily nutrient recommendation includes an estimated daily caloric intake recommendation.
Clause 69. The method of clause 67, wherein the daily nutrient recommendation includes at least one of a daily carbohydrate intake recommendation, a daily fiber intake recommendation, a daily protein intake recommendation, a daily fat intake recommendation, a daily water intake recommendation, a daily vitamin intake recommendation and a daily mineral intake recommendation.
Clause 70. The method of clause 67, wherein the daily nutrient recommendation is based at least in part on the subject's age, the subject's height, the subject's weight and the subject's body mass index.
Clause 71. The method of clause 67, further comprising receiving, by communications circuitry of the reader device, data indicative of one or more heart rate measurements, and wherein the determination to adjust the daily nutrient recommendation is further based on the one or more heart rate measurements.
Clause 72. The method of clause 67, further comprising receiving, by the communications circuitry of the reader device, data indicative of one or more hydration level measurements, and wherein the determination to adjust the daily nutrient recommendation is further based on the one or more hydration level measurements.
Clause 73. The method of clause 39, wherein the at least one analyte level comprises a ketone level.
Clause 74. The method of clause 67, wherein the at least one analyte level comprises a ketone level.
Clause 75. The method of clause 74, further comprising determining and displaying one or more times of a day when a ketosis target threshold is not met, and displaying one or more recommendations for achieving the ketosis target threshold.
Clause 76. The method of clause 69, further comprising displaying a recommendation for a diet modification.

## Claims

1. A system for monitoring glucose levels, the system comprising:
a glucose sensor comprising a portion configured to be placed in fluid contact with a bodily fluid of a user to sense signals indicative of glucose levels of the user;
sensor electronics coupled to the glucose sensor and comprising:
one or more processors,
memory coupled with the one or more processors, wherein the memory stores instructions that when executed by the one or more processors, causes the one or more processors to determine glucose data based on the signals from the glucose sensor, and
wireless communication circuitry configured to communicate the glucose data according to a Bluetooth communication protocol; and
a reader device comprising:
a display;
one or more processors;
a transceiver configured to receive the glucose data from the sensor electronics;
a memory coupled to the processor and storing instructions that when executed by the processor cause the processor to:
determine a glucose curve associated with an ingested food based on the glucose data;
determine a numerical score for the glucose curve associated with the ingested food, wherein the numerical score is based on at least an area under the glucose curve and a length of the glucose curve;
determine a cumulative score by adding a plurality of numerical scores over a predetermined time period; and
display the cumulative score and a target score

2. The system of claim 1, wherein the glucose data comprises one or more glucose metrics.

3. The system of claim 1, wherein the processor is further caused to receive user input of information associated with the ingested food.

4. The system of claim 1, wherein the processor is configured to automatically detect the ingested food based on an excursion of the glucose data outside of a target range.

5. The system of claim 1, wherein the cumulative score comprises a sum of the numerical scores in a day.

6. The system of claim 1, wherein the numerical score is further based on a slope of the glucose curve.

7. The system of claim 1, wherein the numerical score comprises a whole number.

8. The system of claim 1, wherein the processor is further caused to output a tip on the display for helping the user to meet the target score.

9. A method for monitoring glucose levels using a glucose sensor, wherein the method comprises:
determining a glucose curve associated with an ingested food based on glucose data received from sensor electronics coupled to the glucose sensor, wherein the glucose sensor comprises a portion configured to be placed in fluid contact with a bodily fluid of a user to measure glucose levels in the bodily fluid;
determining a numerical score for the ingested food, wherein the numerical score is based on at least an area under the glucose curve and a length of the glucose curve;
determining a cumulative score by adding the numerical scores for a plurality of glucose curves associated with ingested foods in a predetermined period of time; and
outputting, on a display of a reader device in wireless communication with the sensor electronics coupled to the glucose sensor, the cumulative score and a target score.

10. The method of claim 9, wherein the glucose data comprises one or more glucose metrics.

11. The method of claim 9, further comprising receiving user input of information associated with the ingested food.

12. The method of claim 9, wherein the cumulative score comprises a sum of the numerical scores in a day.

13. The method of claim 9, wherein the numerical score is further based on a slope of the glucose curve.

14. The method of claim 9, wherein the numerical score comprises a whole number.

15. The method of claim 9, further comprising displaying a tip for helping the user to meet the target score.
